# EUROPEAN PATENT APPLICATION

(11) **EP 1 777 216 A1**
(43) Date of publication of application: **25.04.2007**
(21) Application number: 05020916.2
(22) Date of filing: 26.09.2005
(51) Int. Cl.: C07C 315/02, C07C 317/46

(54) **A process for the preparation and purification of bicalutamide**

(71) Applicant: SOLMAG S.p.A., 26837 Mulazzano LO (IT)
(72) Inventor: Pizzocaro, Francesco, 20024 Garbagnate (MI) (IT); Pizzocaro, Roberta, 20024 Garbagnate (MI) (IT)
(74) Representative: Minoja, Fabrizio

(57) **Abstract**

The invention relates to a process for the preparation of bicalutamide (I) 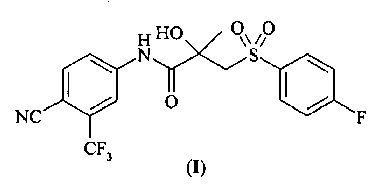 which comprises oxidizing N-[4-cyano-3-(trifluoromethyl)phenyl]-3-[(4-fluorophenyl)thio]-2-hydroxy-2-methyl-propanamide (II) 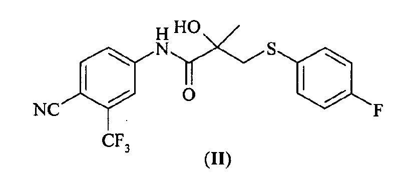 in a solution of peracetic acid in acetic acid and an aromatic solvent. The preparation of **(II)** from 4-fluorothiophenol and 4-cyano-N-(2,3-epoxy-2-methylpropionyl)-3-trifluoromethylaniline is also disclosed.

## Description

### Field of the Invention

The present invention provides a process for the preparation and purification of bicalutamide.

### Background of the invention

Bicalutamide (N-(4-cyano-3-trifluoromethylphenyl)-3-(4-fluorophenylsulfonyl)-2-hydroxy-2-methyl-propionamide) **(I)** 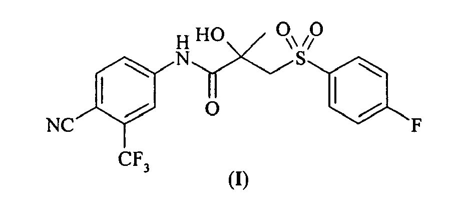 is an antiandrogen, antineoplastic, and anticancer compound, disclosed in EP 100172. The synthesis reported in this patent and also in J. Med. Chem. 31, 954-959 (1988) comprises oxidizing N-[4-cyano-3-(trifluoromethyl)phenyl]-3-[(4-fluorophenyl)thio]-2-hydroxy-2-methyl-propanamide **(II)** 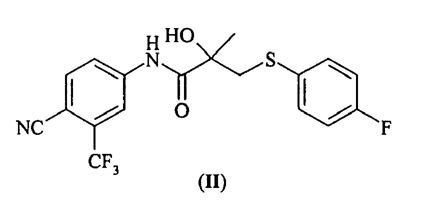 with m-choloroperbenzoic acid in methylene chloride.

Compound (**II**) is in turn synthesised by reaction of 4-cyano-N-(2,3-epoxy-2-methylpropionyl)-3-trifluoromethylaniline **(III)** 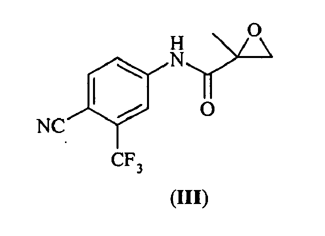 with sodium 4-fluorothiophenol **(IV)** 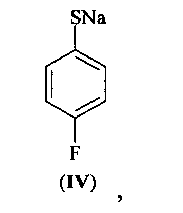 which is prepared from the corresponding thiol by treatment with sodium hydride in anhydrous THF. This procedure is attractive in that it envisages the use of commercially available reagents, but it suffers from some disadvantages: apart from being expensive, m-chloroperbenzoic acid is unstable and can violently decompose, therefore it is not suitable for industrial processes; purification of bicalutamide from the m-chlorobenzoic acid which forms in the reaction is sometimes troublesome, and requires several additional operations, with remarkable increase in costs; halogenated solvents like methylene chloride are harmful, and their use is questioned due to their potential environmental impact; the reaction of sodium hydride with compounds having acid protons, like 4-fluorothiophenol, generates one molar equivalent of hydrogen and the reaction temperature must be strictly controlled, therefore the reaction is difficult to scale up.

WO 01/00608 reports other oxidative processes for transforming compound **(II)** into compound **(I),** which comprise the use of Oxone^{®} (a combination of potassium hydrogen persulfate/potassium hydrogen sulfate/potassium sulfate) or concentrated aqueous hydrogen peroxide solution, in the presence of a salt of a metal belonging to the vanadium or chromium group, in a biphasic system with dichlorometane, and in the presence of a phase transfer catalyst. However, these processes still comprise the use of an expensive reagent (Oxone^{®}) and a hazardous solvent (methylene chloride).

Attempts to replace *m*-choloroperbenzoic acid in the oxidation of **(II)** to bicalutamide are reported for instance in WO 02/24638 and US 2003/0191337. WO 02/24638 discloses a process comprising the use of aqueous hydrogen peroxide and trifluoroacetic anhydride. However, trifluoroacetic anhydride is expensive, corrosive and hygroscopic and must be used at low temperatures, for these reasons it is not suitable for industrial production.

US 2003/0191337 teaches to oxidize **(II)** to **(I)** with mono-perphthalic acid, prepared from aqueous hydrogen peroxide and phthalic anhydride. Alternatively, a concentrated aqueous hydrogen peroxide solution, in the presence of sodium tungstate and a phase transfer catalyst can be used (similarly to what disclosed in WO 01/00608, but with the addition of phenylphosphonic acid). The use of mono-perphthalic acid makes the process expensive for the following reasons: first, mono-perphthalic acid cannot be easily found on the market; second, it cannot be stored; third, the phthalic acid which forms in the reaction must be removed from the reaction mixture and from the product, which requires additional work-up.

As far as purification is concerned, bicalutamide form I (bicalutamide exists in at least two polymorphs) is usually crystallised from ethyl acetate by addition of hexanes or heptanes as described, for instance, in US 2004/0133031. Form I can also be obtained as disclosed in J. Med. Chem. 31, 954-959 (1988), i.e. in ethyl acetate-petroleum ether. The X-ray diffraction data of form I are for instance reported in WO 2004/02021. It is also widely known in the art that the same crystalline form must be prepared in order to maintain the same physical properties in the final drug. However, petroleum ether, hexanes and heptanes are highly inflammable and have a relatively low flash-point, therefore are not safe from the industrial standpoint.

In view of the above, there is still the need for a process for the production and crystallization of bicalutamide suitable for industrial scale.

### Brief description of the drawing

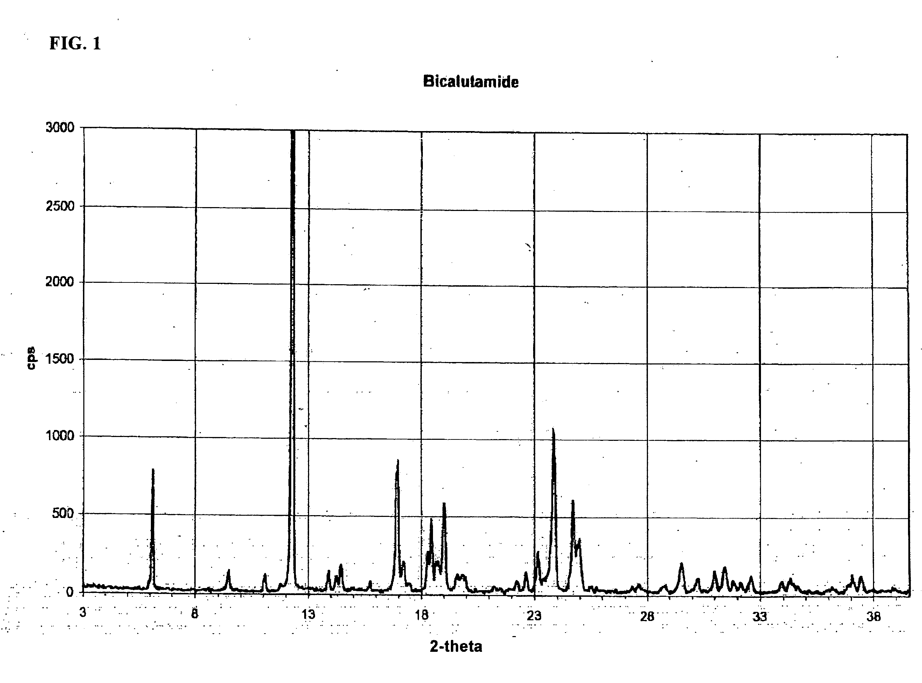

FIG. 1 shows a representative powder X-ray diffraction pattern of Bicalutamide form I, as obtained according to the present invention.

X-ray diffraction patterns were measured with an automated diffractometer θ/θ Ital Structures with CuKr radiation.

### Detailed description of the invention

It has now been found that the above-mentioned drawbacks can be overcome using peracetic acid in acetic acid as the oxidizing agent and by using sodium methylate to prepare compound (**IV**). It has also been found that bicalutamide form I can be conveniently obtained by crystallising crude bicalutamide from a mixture of ethyl acetate and an aromatic solvent.

Accordingly, in a first aspect the invention provides a process for the preparation of bicalutamide (**I**) 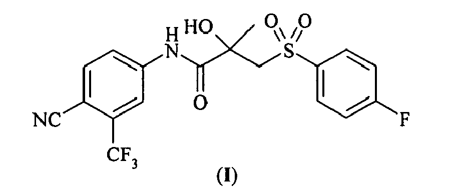 which comprises oxidizing N-[4-cyano-3-(trifluoromethyl)phenyl]-3-[(4-fluorophenyl)thio]-2-hydroxy-2-methyl-propanamide **(II)** 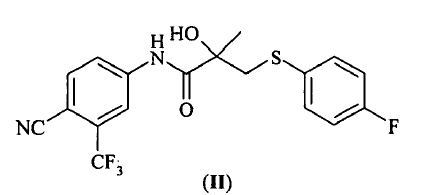 with peracetic acid in acetic acid. The conversion of compound **(II)** into compound (**I**) is carried out in an aromatic solvent, preferably selected from toluene, *o-, m-* and *p*-xylene, the most preferred solvent being toluene. Most preferably, the reaction is carried out in a 25% solution of peracetic acid in acetic acid, in toluene as the solvent, at a temperature ranging from -20 to 100°C, preferably from 0°C to 50°C, most preferably from 0°C to 30°C, for a time of at least ten minutes, preferably for a time ranging from one hour to two days, most preferably from one hour to one day.

In a further aspect, the invention relates to the preparation of compound (**II**) by:
a. treating 4-fluorothiophenol with a base selected from the group consisting of sodium hydroxide, sodium carbonate, sodium methylate, sodium ethylate and sodium *t*-butylate in a protic or aprotic solvent to give sodium 4-fluorothiophenol (**IV**) 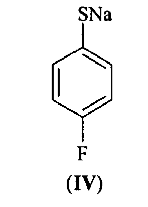 and
b. reacting **(IV)** with 4-cyano-N-(2,3-epoxy-2-methylpropionyl)-3-trifluoromethylaniline **(III)**

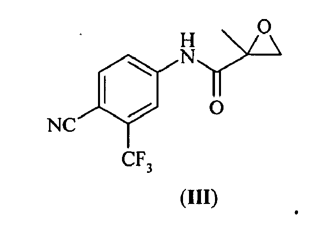

The protic solvents used for the preparation of compound **(IV)** are preferably selected from the group of methanol, ethanol, or *t*-butanol, while the aprotic solvents are preferably toluene and THF. The reaction is usually carried out at a temperature ranging from 0 to 100°C, preferably from room temperature to 80°C.

Preferred bases are sodium methylate and sodium ethylate. Preferred solvents are methanol, ethanol, or, among aprotic solvents, toluene is most preferred. According to a preferred embodiment, compound **(IV)** is directly submitted to the following step, without isolation. In this case, compound **(IV)** is prepared in the same solvent as in the subsequent step, preferably toluene, removing by distillation of any volatiles, like methanol, ethanol, that may have formed in the reaction.

The reaction of compound **(IV)** with compound **(III)** can be carried out, for example, in toluene, *o-, m-,* and *p*-xylene, at a temperature ranging from room temperature to 150°C, for a time ranging from 10 minutes to a few days. The preferred solvent is toluene at a temperature ranging from room temperature to 100°C, preferably from 40°C to 80°C. Preferably, the reaction is carried out for a time ranging from 10 minutes to one day, more preferably from 1 hour to 8 hours, most preferably from 1 to 6 hours. According to a preferred embodiment, when toluene is used as the solvent, after an optional aqueous work-up the toluene solution containing compound **(II)** can be directly used for the oxidation of **(II)** to bicalutamide.

In a still further aspect, the invention provides a process for crystallising bicalutamide from mixtures of an aromatic solvent and ethyl acetate. Preferred crystallization conditions include refluxing crude bicalutamide in a mixture of ethyl acetate and an aromatic solvent selected from toluene and o-, *m-,* and *p*-xylene, to obtain complete dissolution, and then allowing the compound to crystallize by cooling to a temperature ranging from -30°C to room temperature, for a suitable time, comprised between few hours to few days. Preferred crystallisation conditions include refluxing crude bicalutamide in a mixture consisting of ethyl acetate/toluene in ratios ranging from 1:1 to 9:1, preferably ranging from 6:4 to 8:2, most preferably in a 7:3 ratio, until complete dissolution, optionally filtering the solution, and then cooling it to a temperature comprised between 0°C and 40°C, most preferably between 10°C and 20°C. The resulting suspension is then stirred for a suitable time, preferably from an hour to a few days, more preferably from two hours to 24 hours, thereafter bicalutamide is isolated by filtration, optionally washed and then dried at a temperature comprised between room temperature and 100°C, preferably between room temperature and 80°C, most preferably between 40°C and 60°C.

The present invention is advantageous in that, besides the fact that peracetic acid is commercially available, storable, and relatively safe, its by-product (acetic acid) is very easy to remove. Moreover, the use of the above-listed bases in the preparation of compound **(IV)** allows to avoid hydrogen formation and to carry out the whole process without isolating compounds **(IV)** and **(II),** since they are obtained in a satisfactory purity.

The following examples illustrate the invention in greater detail.

### EXAMPLES

### Example 1

### N-[4-cyano-3-(trifluoromethyl)phenyl]-3-[(4-fluorophenyl)thio]-2-hydroxy-2-methyl-propanamide

74 g of 4-cyano-N-(2,3-epoxy-2-methylpropionyl)-3-trifluoromethylaniline was added to sodium 4-fluorothiophenol (55 g) in toluene (500 ml). The mixture was heated to 60-70°C and stirred for two hours, then cooled to about 25-30°C and added with water (150 ml). The organic layer containing the title product was separated and used as such for the following step.

### Example 2

### N-[4-cyano-3-(trifluoromethyl)phenyl]-3-[(4-fluorophenyl)sulfonyl]-2-hydroxy-2-methyl-propanamide

650 ml of the solution obtained according to example 1 was cooled to 5-10°C. 242 g of a 25% w/w solution of peracetic acid in acetic acid was added dropwise, maintaining the temperature at about 20°C. After completion of the addition, the mixture was stirred overnight at room temperature. The mixture was cooled to about 10°C and treated with a sodium hydrosulfite solution in water (350 ml). After one hour stirring the suspension was filtered, washed first with toluene and then with water. After drying, 105 g of crude title compound was obtained.

### Example 3

### Crystallization of N-[4-cyano-3-(trifluoromethyl)phenyl]-3-[(4-fluorophenyl)sulfonyl]-2-hydroxy-2-methyl-propanamide

105 g of crude bicalutamide obtained as described in example 2 was suspended in 640 ml of a 7:3 ethyl acetate/toluene mixture. The suspension was refluxed until complete dissolution. The solution was then cooled to 12-15°C and stirred overnight. The crystalline precipitate was filtered, thoroughly washed with toluene, then dried at 60°C until constant weight. 82 g of pure bicalutamide was obtained. The X-ray diffraction pattern of the product is reported in fig. 1.

### Example 4

### Preparation of sodium 4-fluorothiophenol

A mixture of sodium methylate (20 g) and 4-fluorothiophenol (47 g) in toluene was heated to 60-70°C and stirred for three hours. About 100 ml of the solvent was distilled off, and the remaining mixture was cooled to room temperature. The resulting solution was used as described in example one.

### Example 5

### N-[4-cyano-3-(trifluoromethyl)phenyl]-3-[(4-fluorophenyl)sulfonyl]-2-hydroxy-2-methyl-propanamide

A mixture of sodium methylate (20 g) and 4-fluorothiophenol (47 g) in toluene was heated to 60-70°C and stirred for three hours. After distillation of about 100 ml of the solvent, the remaining mixture was cooled to room temperature. 74 g of 4-cyano-N-(2,3-epoxy-2-methylpropionyl)-3-trifluoromethylaniline was added to the solution, and the resulting mixture was heated to 60-70°C and stirred for two hours. The mixture was then cooled to about 25-30°C and water (150 ml) was added. The organic layer was separated and cooled to 5-10°C. 242 g of a 25% w/w solution of peracetic acid in acetic acid was added dropwise, maintaining the temperature at about 20°C. When the addition was complete, the mixture was stirred overnight at room temperature. The mixture was cooled to about 10°C and treated with a sodium hydrosulfite solution in water (350 ml). After one hour stirring, the suspension was filtered, washed with toluene and then with water. After drying, 105 g of crude title compound was obtained. The crude product was purified by crystallization as described in example 3.

## Claims

1. A process for the preparation of bicalutamide (**I**) 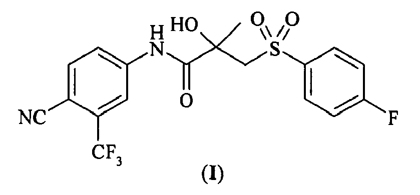 which comprises oxidizing N-[4-cyano-3-(trifluoromethyl)phenyl]-3-[(4-fluorophenyl)thio]-2-hydroxy-2-methyl-propanamide **(II)** 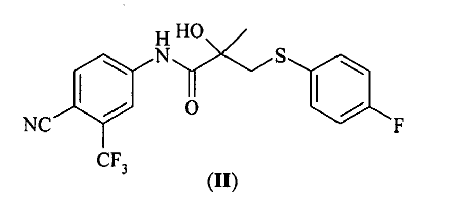 in a solution of peracetic acid in acetic acid and an aromatic solvent.

2. The process according to claim 1 wherein the solvent is selected from toluene, o-, *m-,* and *p*-xylene.

3. The process according to claim 2 wherein the solvent is toluene.

4. The process according to claim 1 to 3 wherein N-[4-cyano-3-(trifluoromethyl)phenyl]-3-[(4-fluorophenyl)thio]-2-hydroxy-2-methyl-propanamide **(II)** is prepared by
a. treating 4-fluorothiophenol with a base selected from the group consisting of sodium hydroxide, sodium carbonate, sodium methylate, sodium ethylate and sodium *t*-butylate in a protic or aprotic solvent to give sodium 4-fluorothiophenol (**IV**) 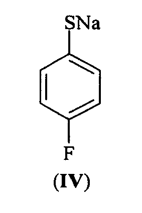
b. and reacting **(IV)** with 4-cyano-N-(2,3-epoxy-2-methylpropionyl)-3-trifluoromethylaniline **(III)** 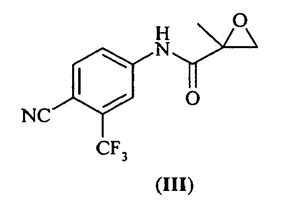 in a solvent selected from toluene, *o-, m-,* and *p*-xylene.

5. The process according to claim 4, wherein the base is sodium methylate or sodium ethylate.

6. The process according to claim 4 wherein the protic solvent for step a) is selected from the group consisting of water, methanol, ethanol and *t*-butanol.

7. The process according to claim 6 wherein the solvent is methanol or ethanol.

8. The process according to claim 4 wherein the aprotic solvent for step a) is toluene or tetrahydrofuran.

9. The process according to claim 8 wherein the solvent is toluene.

10. The process according to claim 4 wherein the solvent for step b) is toluene.

11. The process according to claims 9 and 10 which is carried out without isolating compounds (**IV**) and (**II**).

12. The process according to any one of claims 1-11 further comprising recrystallising bicalutamide from ethyl acetate and an aromatic solvent.

13. The process according to claim 12 wherein the aromatic solvent is selected from toluene, *o-, m-*, and *p*-xylene.

14. The process according to claim 12 wherein the aromatic solvent is toluene.
